(19) Europäisches Patentamt
European Patent Office
Office européen des brevets

(11) Publication number: **0 120 359**
**A1**

# (12) EUROPEAN PATENT APPLICATION

(21) Application number: 84102433.4

(22) Date of filing: 07.03.84

(51) Int. Cl.$^3$: C 07 D 333/38
A 61 K 31/38

(30) Priority: 23.03.83 IT 2023983

(43) Date of publication of application:
03.10.84 Bulletin 84/40

(84) Designated Contracting States:
AT BE CH DE FR GB IT LI LU NL SE

(71) Applicant: YASON S.r.l
Via Pisacane, 34/A
I-20129 Milan(IT)

(72) Inventor: Quadro, Giuseppe
Via Pisacane, 34/A
I-20129 Milan(IT)

(74) Representative: Bianchetti, Giuseppe
Studio Consulenza Brevettuale Via Senato 24
I-20121 Milan(IT)

## (54) Tetrahydrothiophenone derivative active in respiratory system affections.

(57) N-(2-Thenoyl)-N-(2-keto-2,3,4,5-tetrahydro-thiophene-3-yl)amine of formula I

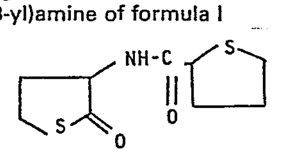

I

is a valuable bronchosecretogogue, antitussive and anti-inflammatory agent.

It may be prepared by reacting homocysteine thiolactone hydrochloride with a reactive derivative of 2-thiofenecarboxylic acid.

The compound of the invention may be formulated with conventional pharmaceutically acceptable carriers or diluents, to provide a pharmaceutical composition.

EP 0 120 359 A1

Croydon Printing Company Ltd.

"Tetrahydrothiophenone derivative active in respiratory system affections"

The present invention relates to N-(2-thenoyl)-N-(2-keto-2,3,4,5-tetrahydro-thiophene-3-yl)amine of formula I

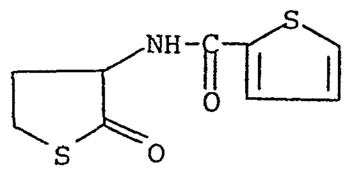

I

to a process for preparing it and to pharmaceutical compositions containing it.

This compound, which will be hereinafter named YS-869 for sake of shortness, is a valuable agent in the treatment of respiratory system affections such as bronchitis, tracheobronchitis, bronchopneumonia, asthma, enphysema and in the symptomatic therapy of every kind of acute and chronic cough.

YS-869 in fact exhibits excellent mucusregulating, bronchosecretogogue and antitussive activities.

The compound of the invention has another advantageous activity, that is a potent antiinflammatory action, comparable to the one of acetylsalicylic acid, and moreover practically no gastrolesivity degree, by far lower than the one of acetylsalicylic acid.

Such an activity, in addition to the above mentioned ones, allows to carry out a complete and ef

fective treatment of respiratory system diseases with inflammatory condition.

The compound of the invention is prepared by reacting homocysteine thiolactone hydrochloride with a 2-thiophenecarboxylic acid reactive derivative, such as an acyl chloride or anhydride.

The reaction is effected in the presence of acid-binding agents, preferably bases such as alkali hydroxides previously added to the homocysteine thiolactone hydrochloride in the form of aqueous solutions.

The acylating reaction is preferably carried out at temperatures ranging from 0° to +20°C.

As the reactive derivative an acyl chloride, dissolved in aromatic hydrocarbons, is employed: the addition of the chloride to the reaction mixture is carried out at temperatures between 0° and +5°C. After completion of the reaction, the product may be recovered from the reaction mixture by conventional means.

This compound may be further purified by such conventional techniques as column chromatography or recrystallization.

The preparation of the compound of the invention is further illustrated by the following nonlimiting example.

EXAMPLE

16.8 G of homocysteine thiolactone hydrochloride and 4.35 g of NaOH were dissolved in 150 ml of

water, with stirring.

The reaction mixture was then cooled to 0°C and 18 g of the acyl chloride of 2-thiophene-carboxylic acid dissolved in 15 ml of benzene was added. The resulting mixture was stirred for 1 hour below 20°C, then the reaction mixture was diluted with 50 ml of petroleum ether.

The resulting residue was filtered, dissolved in dichloromethane and washed with a hydrogenocarbonate solution.

9 G of pure product was obtained, showing single spot in TLC.

The product was recrystallized from 1:1 dichloromethan/diethyl ether mixture. M.p. 127-129°C. The compound is soluble in common organic solvents.

Elemental analysis for $C_9H_9NO_2S_2$ (M.W. = 227.29)

Calculated % C = 47.55; H = 3.99; N = 6.15

Found % C = 47.5; H = 3.92; N = 6.10.

The structure of the compound is confirmed by the spectroscopic data.

I.R. Spectrum (in nujol mull, the values of the absorption bands are given in $cm^{-1}$):

| | |
|---|---|
| stretch N-H | 3250 |
| stretch C=O thiolactone | 1680 |
| stretch C=O amide | 1625 |

$H^1$ NMR Spectrum (registered in $CDCl_3$ + DMSO mixture 80:20; internal standard TMS; the values of chemical shifts of protons are given in $\delta$):

1.8 - 2.5 (m, 2H, S-$CH_2$-$\underline{CH_2}$);
2.9 - 3.35 (m, 2H, S-$\underline{CH_2}$-$CH_2$);
4.3 - 4.9 (m, 1H, CO-CH-NH);
6.7 - 7.65 (m, 3H aromatic);
8.3 - 8.6 (d, 1H, NH mobile).

The biological properties of the compound of the invention are described hereinbelow.

Pharmacologic activity

Bronchosecretogogue activity

The bronchosecretogogue activity of YS-869 was evalued by the test of Mawatari in Sprague-Dawley rats (Mawatari H. "Experimental Studies on the expectorant action of several drugs" - Kagoshima Daigaken Igaken Zasshi. 27, 561, 1976), in comparison with known drugs.

This method consists in determinating the amount of sodium fluoride eliminated with the fluid in the respiratory tract.

YS-869 at a dosage of 500 mg/kg exhibits a bronchosecretogogue activity higher than the one of Ambroxol and nearly equal to the one of S-carboxymethyl-cysteine, causing in the treated rats a 50% increase of the NaF elimination in the secretion with respect to the control group (see Table I).

TABLE I - Bronchosecretogogue activity - Mawatari test in rat

| TREATMENT | DOSE mg/kg i.p. | N° animals | ANIMALS WEIGHT (g) | Fl Na | | Significance level-Student t |
|---|---|---|---|---|---|---|
| | | | | µg/ml | % increase versus contr. | |
| CONTROL | - | 15 | 105.3 ± 3.33 | 0.28 ±0.027 | - | - |
| AMBROXOL | 50 | 15 | 97.0 ± 2.80 | 0.36 ± 0.037 | 28.6 | Not significant |
| S-CARBOXY-METHYL-CYSTEINE | 500 | 13 | 101.9 ± 3.60 | 0.41 ± 0.036 | 46.4 | $p \leq 0.01$ |
| YS-869 | 500 | 13 | 102.7 ± 3.56 | 0.42 ± 0.0450 | 50.0 | $p < 0.01$ |

Antitussive activity

The antitussive activity of YS-869 was evalued by the citric acid test in the rabbit (Boura et al., Prog. Brit. Pharmac. Soc. Communications, April 1970), in comparison with codeine.

Such method consists in counting the coughs strokes caused in the animal by the nebulization of a citric acid solution.

YS-869 at a dosage of 100 mg/kg decreased by 21.5% the coughs in the treated animals with respect to the control group, whilst the codeine caused a 70.4% decrease. (See Table II).

TABLE II - Antitussive activity - Citric acid induced cough in rabbit.

| TREATMENT | DOSE mg/kg i.p. | N° animals | $\bar{X}$ weight grams | Cough strokes in 10 min. | % inhibition versus control |
|---|---|---|---|---|---|
| CONTROL | - | 10 | 279.0 $\pm$7.37 | 22.3 $\pm$1.14 | - |
| CODEINE | 25 | 10 | 253.8 $\pm$26.44 | 6.6*** $\pm$1.06 | 70.4 |
| YS-869 | 100 | 10 | 294.0 $\pm$5.26 | 17.5** $\pm$1.14 | 21.5 |

Student test:

* $p < 0.05$
** $p < 0.01$
*** $p < 0.001$

Antiinflammatory activity

The antiinflammatory activity of YS-869 in comparison with acetylsalicylic acid was evaluated by the test of oedema induced by carrageening injection in rat paw (Winter, Rissly, Ness.; Proc. Soc. Exp. Biol. Med., 111, 544-47 - 1962).

The oedema inhibiting activity, turned out to be 20.26%, at a dosage of 252 mg/kg per os of YS-869, in comparison with a 26.94% activity of acetylsalicylic acid at the equimolecular dosage of 200 mg/kg.

The results are reported in Table III.

TABLE III - Antiinflammatory activity - Carrageenin oedema in rat.

| TREATMENT | DOSE mg/kg p.o. | N° animals | Paw volume ml/10-M±E.S. | | | | | | AREA | |
|---|---|---|---|---|---|---|---|---|---|---|
| | | | Basal | 2 h | 4 h | 6 h | 8 h | 24 h | M±E.S. | % Inhibition versus control |
| CONTROL | - | 10 | 35.4 ±0.9 | 53.0 ±1.9 | 55.0 ±1.4 | 53.8 ±1.3 | 49.1 ±1.3 | 43.5 ±1.1 | 207.2 ±11.2 | - |
| Acetylsalicylic acid | 200 | 10 | 36.1 ±1.1 | 43.5 ±1.5 | 49.3 ±2.0 | 51.4 ±1.7 | 51.5 ±2.0 | 42.8 ±1.7 | 151.38 ±13.1 | 26.94 |
| YS-869 | 252 | 10 | 36.8 ±1.0 | 48.2 ±1.2 | 54.1 ±2.3 | 52.1 ±2.9 | 51.4 ±2.5 | 41 ±1.4 | 165.21 ±13.1 | 20.26 |

Gastro-injuring activity

The gastro-injuring activity was determined by administering rats with equimolecular dosages of YS-869 and acetylsalicylic acid (252 mg/kg and 200 mg/kg respectively).

The gastric ulcers average size was 1.8 mm in the animals administered with YS-869 and 27.7 mm in the control group treated with acetylsalicylic acid.

The results are reported in Table IV.

TABLE IV - GASTRO-INJURING ACTIVITY

| TREATMENT | DOSE mg/kg p.o. | N° animals | $\bar{X}$ weight g | ULCERS mm |
|---|---|---|---|---|
| CONTROL | - | 10 | $129.3 \pm 4.85$ | $1.1 \pm 0.64$ |
| Acetyl-salicylic acid | 200 | 10 | $130.7 \pm 6.90$ | $27.7 \pm 6.37$*** |
| YS-869 | 252 | 10 | $125 \pm 5.97$ | $1.8 \pm 1.08$ |

Student test ***$p < 0.001$

The invention also provides pharmaceutical compositions containing, as active ingredient, prefixed and therapeutically effective amounts of YS-869, in admixture with a pharmaceutically acceptable carrier or diluent to be administered orally, rectally, parenterally or by inhalatory route.

Examples of such formulations are tablets, capsu

les, pills, powders, granules, syrups, suppositories, phials, ampoules,aerosols, possible sustained release dosage forms, obtained for instance by microincapsulation, together with suitably excipients commonly used in pharmaceutical technique.

The preferred dose of YS-869 will vary, depending upon the weight of the patient and the severity of the disease; generally it will range from 50 to 1000 mg.

CLAIMS

1. N-(2-Thenoyl)-N-(2-keto-2,3,4,5-tetrahydro-thio phene-3-yl)amine of formula I

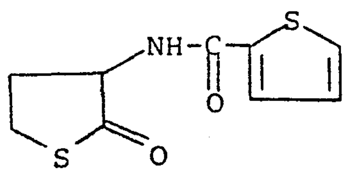

I

2. A process for preparing compound I, which process comprises reacting homocysteine thiolactone hydrochloride with a reactive derivative of 2-thio phene-carboxylic acid.

3. A process according to claim 2, wherein said reactive derivative of 2-thiophene-carboxylic acid is the acyl chloride.

4. A process according to claim 2 and 3, wherein said reaction is carried out in the presence of an acid-binding agent.

5. A process according to claim 4, wherein said acid-binding agent is an inorganic base, such as an alkali hydroxide.

6. A process according to claims 2-5, characterized in that the 2-thiophene-carboxylic acid acyl ch loride dissolved in aromatic hydrocarbons is added at temperatures ranging from 0 to +5°C to a water solution of sodium hydroxide and homocysteine thio lactone hydrochloride and that the reaction is car ried out at temperatures ranging from 0 to +20°C.

7. Pharmaceutical compositions having antitussive,

mucusregulating and antiinflammatory activity, wherein said compositions comprise, as active ingredient, compound according to claim 1.

8. Pharmaceutical compositions according to claim 7, in form of capsules, tablets, pills, syrups, suppositories, solutions.

CLAIMS

1. A process for preparing N-(2-thenoyl)-N-(2-keto-2,3,4,5-tetrahydro-thiofene-3-yl)amine of formula I

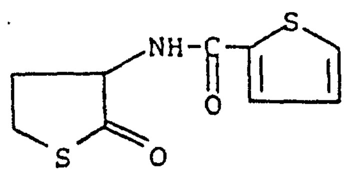

I

which process comprises reacting homocysteine thio lactone hydrochloride with a reactive derivative of 2-thiophene-carboxylic acid.

2. A process according to claim 1, wherein said reactive derivative of 2-thiophene-carboxylic acid is the acyl chloride.

3. A process according to claim 1 and 2, wherein said reaction is carried out in the presence of an acid-binding agent.

4. A process according to claim 3, wherein said acid-binding agent is an inorganic base, such as an alkali hydroxide.

5. A process according to claims 2-4, characterized in that the 2-thiophene-carboxylic acid acyl chloride dissolved in aromatic hydrocarbons is added at temperatures ranging from 0 to +5°C to a water solution of sodium hydroxide and that the reaction is carried out at temperatures ranging from 0 to +20°C.

European Patent Office

# EUROPEAN SEARCH REPORT

0120359
Application number

EP 84 10 2433

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | CHEMICAL ABSTRACTS, vol. 89, no. 25, 18th December 1978, page 577, no. 215212u, Columbus, Ohio, US; & ES - A - 457 424 (LABORATORIOS ROGER S.A.) 16-02-1978 * Abstract * | 1-8 | C 07 D 333/38<br>A 61 K 31/38 |
| | --- | | |
| X,Y | FR-A-2 265 355 (J. BLUM) * Claims * | 1-8 | |
| | --- | | |
| Y | CHEMICAL ABSTRACTS, vol. 89, no. 3, 17th July 1978, page 694, no. 24801a, Columbus, Ohio, US; & ES - A - 447 648 (LABORATORIOS ROGER S.A.) 01-06-1977 * Abstract * | 1 | |
| | ----- | | |
| | | | TECHNICAL FIELDS SEARCHED (Int. Cl. 3) |
| | | | C 07 D 333/00<br>A 61 K 31/00 |

The present search report has been drawn up for all claims

| Place of search | Date of completion of the search | Examiner |
|---|---|---|
| THE HAGUE | 21-06-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82